# EUROPÄISCHE PATENTANMELDUNG

(11) **EP 2 369 335 A1**
(43) Veröffentlichungstag der Anmeldung: **28.09.2011**
(21) Anmeldenummer: 11151505.2
(22) Anmeldetag: 20.01.2011
(51) Int. Cl.: G01N 33/00

(54) **Vorrichtung zur Bestimmung eines Quecksilbergehalts einer Probe**

(30) Priorität: 17.02.2010 DE 202010002443 U
(71) Anmelder: MWT Mikrowellen Labor Technik AG, 9435 Heerbrugg (CH)
(72) Erfinder: Lautenschläger, Jens, 9435 Heerbrugg/SG (CH); Lautenschläger, Werner, 9435 Heerbrugg/SG (CH)
(74) Vertreter: Rupp, Christian

(57) **Zusammenfassung**

Die Erfindung betrifft eine Vorrichtung zur Bestimmung eines Quecksilbergehalts einer Probe (2); die Vorrichtung weist einen Verbrennungsraum (4) zum Verbrennen der Probe (2) unter Sauerstoff auf, sowie eine erste, mit dem Verbrennungsraum (4) verbundene Zuführungsvorrichtung (6) zum Zuführen des Sauerstoffs in den Verbrennungsraum (4). Weiterhin weist die Vorrichtung eine zweite, mit dem Verbrennungsraum (4) verbundene Zuführungsvorrichtung (8) zum Zuführen der Probe (2) in den Verbrennungsraum (4) auf. Durch die zweite Zuführungsvorrichtung (8) lässt sich eine Bestimmung des Quecksilbergehalts insbesondere auch im Fall einer gasförmigen Probe ermöglichen. Eine gasförmige Probe kann beispielsweise ein Pyrolysegas umfassen, beispielsweise ein Pyrolysegas aus einer ersten Verbrennungsstufe.

## Beschreibung

Die Erfindung betrifft eine Vorrichtung zur Bestimmung eines Quecksilbergehalts einer Probe, wobei die Vorrichtung einen Verbrennungsraum zum Verbrennen der Probe unter Sauerstoff sowie eine mit dem Verbrennungsraum verbundene Zuführungsvorrichtung zum Zuführen des Sauerstoffs in den Verbrennungsraum aufweist.

Quecksilber ist unter den Metallen eines der mobilsten Elemente und verteilt sich somit zunehmend auf der Erdoberfläche und in der Atmosphäre. Die größten Mengen an Quecksilber werden neben vulkanischen Aktivitäten durch den Menschen freigesetzt. Durch die Verbrennung fossiler Brennstoffe und durch thermische Verfahren wie Erzaufbereitung für die Metall- und Stahlproduktion, sowie Verarbeitung von keramischen Rohstoffen usw. wird Quecksilber in Umlauf gebracht. Die gebildeten Quecksilber-Verbindungen werden durch Bioakkumulation in Lebewesen angereichert und gelangen so in Form von hochtoxischem Methylquecksilber, vor allem durch den Verzehr von Fischen und Meeresprodukten, in den Nahrungskreislauf. Somit sind Quecksilberkontrollen in gasförmigen und flüssigen Proben von zunehmender Bedeutung.

Aus der Literatur ("Dem Quecksilber auf der Spur", Lautenschläger, W.; McKinney, C., Laborpraxis-Würzburg, 2004, 28, 6, Seiten 30 bis 32) ist ein System mit der Bezeichnung "DMA-80" bekannt, mit dem der Quecksilbergehalt in flüssigen und festen Proben ohne vorherige chemische Aufbereitung in kurzer Zeit bestimmt werden kann. Hierzu werden die Proben bei hoher Temperatur katalytisch im Sauerstoffstrom verbrannt und das Quecksilber als Goldamalgam angereichert. Anschließend wird das gesammelte Quecksilber durch spontanes Atomisieren spektroskopisch bestimmt. Diese bekannte Vorrichtung weist einen Verbrennungsraum mit einer Öffnung auf, durch die sowohl der Sauerstoff, als auch die Probe zugeführt werden.

Der Erfindung liegt die Aufgabe zugrunde, eine Vorrichtung zur Bestimmung eines Quecksilbergehalts einer Probe anzugeben, die besonders vielseitig einsetzbar ist.

Diese Aufgabe wird gemäß der Erfindung mit der in dem unabhängigen Anspruch genannten Vorrichtung gelöst. Besondere Ausführungsarten der Erfindung sind in den abhängigen Ansprüchen angegeben.

Gemäß der Erfindung ist eine Vorrichtung zur Bestimmung eines Quecksilbergehalts einer Probe vorgesehen, die einen Verbrennungsraum zum Verbrennen der Probe unter Sauerstoff aufweist, sowie eine erste, mit dem Verbrennungsraum verbundene Zuführungsvorrichtung zum Zuführen des Sauerstoffs in den Verbrennungsraum. Weiterhin weist die Vorrichtung eine zweite, mit dem Verbrennungsraum verbundene Zuführungsvorrichtung zum Zuführen der Probe in den Verbrennungsraum auf sowie Mittel zur Erzeugung eines Unterdrucks, die dazu ausgebildet sind, in dem Verbrennungsraum befindliche Verbrennungsgase durch den Unterdruck aus dem Verbrennungsraum zu befördern.

Durch die zweite Zuführungsvorrichtung lässt sich eine Bestimmung des Quecksilbergehalts insbesondere auch im Fall einer gasförmigen Probe ermöglichen. Bei einer gasförmigen Probe kann es sich beispielsweise um ein Pyrolysegas handeln, insbesondere um ein Pyrolysegas aus einer ersten Verbrennungsstufe. Durch die Mittel zur Erzeugung des Unterdrucks lassen sich die bei einer Verbrennung der Probe in dem Verbrennungsraum entstandenen Verbrennungsgase für eine anschließende Analyse besonders effektiv und kontrolliert aus dem Verbrennungsraum befördern.

Vorzugsweise ist die zweite Zuführungsvorrichtung zum Zuführen einer flüssigen und/oder gasförmigen Probe in den Verbrennungsraum ausgebildet. Vorzugsweise ist die Vorrichtung weiterhin zum kontinuierlichen und/oder diskontinuierlichen Zuführen der Probe in den Verbrennungsraum ausgebildet. Durch diese Ausgestaltungen eignet sich die Vorrichtung besonders gut für diverse, unterschiedliche Arten von Proben.

Vorzugsweise weist die Vorrichtung weiterhin eine Gasströmungs-Führungseinrichtung auf, die mit dem Verbrennungsraum verbunden ist und die dazu ausgebildet ist, eine Gasströmung aus dem Verbrennungsraum zu führen, wobei die Mittel zur Erzeugung des Unterdrucks, vorzugsweise in Form einer Vakuumpumpe, dazu ausgebildet sind, die im Verbrennungsraum befindlichen Gase durch den Unterdruck in die Gasströmungs-Führungseinrichtung zu saugen. Auf diese Weise lässt sich ein Verbrennungsgas, das in dem Verbrennungsraum bei der Verbrennung der Probe gebildet worden ist, für eine anschließende Analyse aus dem Verbrennungsraum in die Gasströmungs-Führungseinrichtung transportieren. Auch überschüssiger Sauerstoff lässt sich hierdurch aus dem Verbrennungsraum in die Gasströmungs-Führungseinrichtung transportieren. Außerdem lässt sich durch die Mittel zur Erzeugung des Unterdrucks vermeiden, dass das Verbrennungsgas, nachdem es zur Analyse in die Gasströmungs-Führungseinrichtung transportiert worden ist, in den Verbrennungsraum zurück diffundiert und auf diese Weise die Analyse fehlerbehaftet erfolgt. Ein entsprechender Verlust, der durch die Verbrennungsgasbildung, verbunden mit Druckanstieg und Dichtungsproblemen entstehen könnte, lässt sich somit verhindern.

Vorzugsweise ist die Pumpe dabei weiterhin dazu ausgelegt, die Probe aus der zweiten Zuführungsvorrichtung in den Verbrennungsraum zu saugen oder eine Bewegung der Probe aus der zweiten Zuführungsvorrichtung in den Verbrennungsraum zu unterstützen. Hierdurch lässt sich ein Einbringen bzw. Zuführen der Probe aus der zweiten Zuführungsvorrichtung in den Verbrennungsraum besonders effektiv gestalten. Insbesondere lässt sich auf diese Weise ein möglicher Strömungswiderstand kompensieren oder zumindest verringern, der durch einen in dem Verbrennungsraum angeordneten Katalysator hervorgerufen ist und der einem Einbringen bzw. Zuführen der Probe in den Verbrennungsraum entgegenwirkt.

Vorzugsweise weist die Vorrichtung weiterhin einen Amalgam-Kollektor zum Binden von in dem Verbrennungsraum aus der Probe freigesetztem Quecksilber auf. Das auf diese Weise gesammelte Quecksilber lässt sich nachfolgend Atomisieren und anschließend beispielsweise mit einem Spektrometer quantitativ erfassen. Dabei ist der Amalgam-Kollektor vorzugsweise innerhalb der Gasströmungs-Führungseinrichtung stromabwärts von dem Verbrennungsraum angeordnet, wobei die Mittel zur Erzeugung des Unterdrucks dazu ausgebildet sind, die in dem Verbrennungsraum befindlichen Verbrennungsgase vollständig aus dem Verbrennungsraum in den Amalgam-Kollektor zu saugen. Das bei der Verbrennung der Probe in dem Verbrennungsraum gebildete Verbrennungsgas kann somit - insbesondere nach einem Durchströmen eines in dem Verbrennungsraum angeordneten Katalysators - vorteilhaft dem Amalgam-Kollektor zugeführt werden.

Vorzugsweise umfasst der Verbrennungsraum einen ersten Heizbereich und einen zweiten Heizbereich, wobei der erste Heizbereich zum Verbrennen der Probe ausgebildet ist und der zweite Heizbereich zum Zersetzen von bei dem Verbrennen der Probe entstandenen Verbrennungsgasen ausgebildet ist.

Vorzugsweise ist der Verbrennungsraum durch eine Heizvorrichtung gebildet, die ein beheizbares Rohr aus Quarz oder Keramik umfasst.

Vorzugsweise weist die Vorrichtung weiterhin eine Analysevorrichtung zur quantitativen Erfassung des in dem Verbrennungsraum bei der Verbrennung aus der Probe freigesetzten Quecksilbers auf. Dabei ist weiterhin vorzugsweise die Analysevorrichtung innerhalb der Gasströmungs-Führungseinrichtung stromabwärts von dem Verbrennungsraum angeordnet.

Vorzugsweise ist die Analysevorrichtung eine spektroskopische Vorrichtung, die weiterhin vorzugsweise eine Quecksilberlichtquelle und einen dazugehörigen Detektor mit einem Monochromator umfasst.

Die Erfindung wird im Folgenden anhand von Ausführungsbeispielen und mit Bezug auf die Zeichnungen näher erläutert. Es zeigen:
- Fig. 1: eine Prinzip-Darstellung eines ersten Ausführungsbeispiels einer erfindungsgemäßen Vorrichtung und
- Fig. 2: eine Prinzip-Darstellung eines zweiten Ausführungsbeispiels.

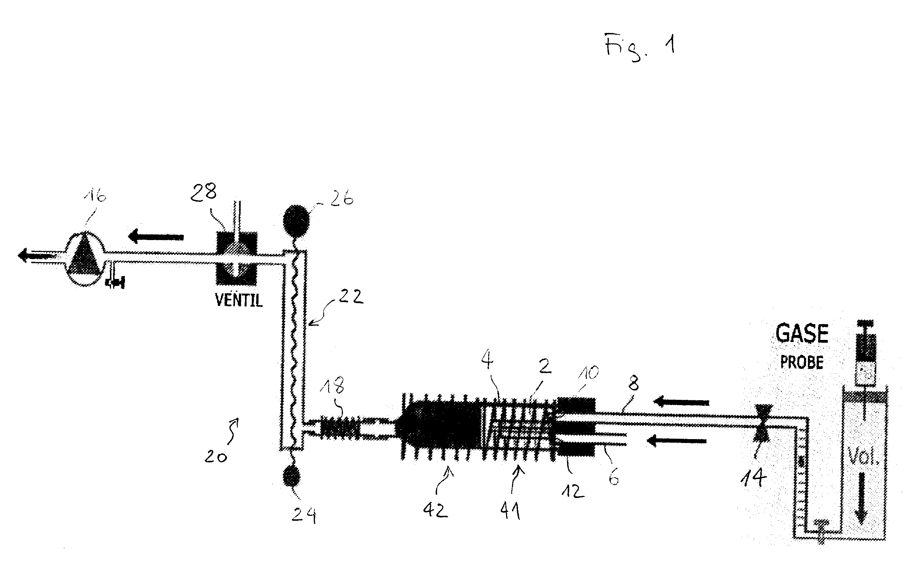

Fig. 1 zeigt ein erstes Ausführungsbeispiel einer erfindungsgemäßen Vorrichtung zur Bestimmung eines Quecksilbergehalts einer Probe 2. Bei der Probe 2 kann es sich allgemein um eine gasförmige oder um eine flüssige Probe handeln. Beispielsweise kann es sich um ein brennbares Gas, um ein Abgas oder um ein Pyrolysegas handeln.

Die Vorrichtung umfasst einen Verbrennungsraum 4 zum Verbrennen der Probe 2 unter Sauerstoff und eine erste Zuführungsvorrichtung 6, die mit dem Verbrennungsraum 4 verbunden ist und die zum Zuführen des Sauerstoffs in den Verbrennungsraum 4 dient.

Weiterhin weist die Vorrichtung eine zweite Zuführungsvorrichtung 8 auf, die mit dem Verbrennungsraum 4 verbunden ist und die zum Zuführen der Probe 2 in den Verbrennungsraum 4 dient.

Der Verbrennungsraum 4 kann durch eine Heizvorrichtung gebildet sein, die ein beheizbares Rohr, vorzugsweise aus Quarz oder Keramik umfasst. Insbesondere kann der Verbrennungsraum 4 im Inneren des beheizbaren Rohrs gebildet sein. Die Heizvorrichtung kann dazu ausgelegt sein, den Verbrennungsraum 4 von außen her elektrisch zu beheizen.

Vorzugsweise ist die Vorrichtung dazu ausgelegt, die Verbrennung der Probe 2 in dem Verbrennungsraum 4 temperaturprogrammiert durchzuführen.

Die zweite Zuführungsvorrichtung 8 kann ein Rohrelement umfassen, das einen kleineren Durchmesser als das beheizbare Rohr der Heizvorrichtung aufweist. Die zweite Zuführungsvorrichtung 8, insbesondere deren Rohrelement, kann an einem Verbindungsort 10 mit dem Verbrennungsraum 4 verbunden sein bzw. an dem Verbindungsort 10 in den Verbrennungsraum 4 einmünden. In der Skizze der Fig. 1 mündet das Rohrelement der zweiten Zuführungsvorrichtung 8 von rechts an dem Verbindungort 10 in die Verbrennungskammer 4.

Vorzugsweise mündet die erste Zuführungsvorrichtung 6 an einem weiteren Verbindungsort 12 in den Verbrennungsraum 4, wobei sich der weitere Verbindungsort 12 von dem zuerst genannten Verbindungsort 10 unterscheidet bzw. der weitere Verbindungsort 12 von dem zuerst genannten Verbindungsort 10 räumlich getrennt ist.

Die zweite Zuführungsvorrichtung 8 ist vorteilhaft derart ausgebildet, dass sie sich zum Zuführen einer flüssigen und/oder gasförmigen Probe in den Verbrennungsraum 4 eignet. Die Vorrichtung lässt sich auf diese Weise insbesondere so ausbilden, dass sie sich für die Bestimmung eines Quecksilbergehalts einer gasförmigen bzw. flüssigen Probe eignet. Dabei ist die zweite Zuführungsvorrichtung 8 weiterhin vorteilhaft derart ausgebildet, dass sie sich zum kontinuierlichen und/oder diskontinuierlichen Zuführen der Probe 2 in den Verbrennungsraum 4 eignet. Hierdurch lässt sich die Vielseitigkeit der erfindungsgemäßen Vorrichtung weiter steigern.

In Fig. 1 ist symbolisch eine Ausgestaltung skizziert, die sich zur Zuführung einer gasförmigen Probe 2 eignet.

Allgemeiner kann die zweite Zuführungsvorrichtung 8 derart ausgebildet sein, dass sich die Probe 2 in einem diskret abgemessenen Volumen in den Verbrennungsraum 4 einspritzen lässt. Die zweite Zuführungsvorrichtung 8 kann alternativ oder ergänzend auch ein Pumpelement aufweisen und dabei derart ausgebildet sein, dass sich die Probe 2 durch das Pumpelement kontinuierlich in den Verbrennungsraum 4 einbringen lässt.

Die zweite Zuführungsvorrichtung 8 kann ein Ventilelement 14 zur Steuerung der Zuführung der Probe 2 in den Verbrennungsraum 4 umfassen. Das Ventilelement 14 kann dabei zum Verschließen und Öffnen des Rohrelements der zweiten Zuführungsvorrichtung 8 vorgesehen bzw. ausgebildet sein.

Weiterhin kann die Vorrichtung eine Gasströmungs-Führungseinrichtung 20 umfassen, die mit dem Verbrennungsraum 4 verbunden ist und die dazu ausgebildet ist, eine Gasströmung aus dem Verbrennungsraum 4 zu führen. Insbesondere kann die Gasströmungs-Führungseinrichtung 20 dazu ausgebildet sein, ein Verbrennungsgas, das bei der Verbrennung der Probe 2 in dem Verbrennungsraum 4 entstanden ist, aus dem Verbrennungsraum 4 zu führen. Ein derartiges Verbrennungsgas enthält möglicherweise ursprünglich in der Probe 2 enthaltenes Quecksilber, das dann im Folgenden quantitativ erfasst werden kann.

Die Gasströmungs-Führungseinrichtung 20 kann strömungstechnisch vorteilhaft mit dem Verbrennungsraum 4 an einer Stelle verbunden sein, die mit Bezug auf einen mittleren Bereich des Verbrennungsraums 4 dem Verbindungsort 10, also der Einmündung der zweiten Zuführungsvorrichtung 8 gegenüberliegt. In der Skizze der Fig. 1 ist die Gasströmungs-Führungseinrichtung 20 in diesem Sinne an der linken Seite mit dem Verbrennungsraum 4 verbunden.

Weiterhin vorteilhaft weist die Vorrichtung eine Pumpe 16 auf, vorzugsweise eine Vakuumpumpe, die dazu ausgebildet ist, ein in dem Verbrennungsraum 4 befindliches, möglicherweise Quecksilber aufweisendes, Gas in die Gasströmungs-Führungseinrichtung 20 zu pumpen bzw. zu saugen. Mit den Ausdrücken "stromabwärts" und "stromaufwärts" seien im vorliegenden Zusammenhang örtliche Bereiche mit Bezug auf die so erzeugte Strömung bezeichnet. Wie beim hier gezeigten Ausführungsbeispiel der Fall, kann die Pumpe 16 in diesem Sinne stromabwärts von dem Verbrennungsraum 4 in der Gasströmungs-Führungseinrichtung 20 angeordnet sein und dazu ausgebildet sein, das Gas aus dem Verbrennungsraum 4 heraus und im Weiteren Verlauf durch die Gasströmungs-Führungseinrichtung 20 hindurch zu saugen.

Vorzugsweise ist die Pumpe 16 auch dazu ausgebildet, die Probe 2 aus der zweiten Zuführungsvorrichtung 8, speziell aus dem Rohrelement der zweiten Zuführungsvorrichtung 8, durch Saugen in den Verbrennungsraum 4 zu transportieren oder zumindest einen Transport bzw. eine Bewegung der Probe 2 in den Verbrennungsraum 4 zu unterstützen. Das Zuführen der Probe 2 in den Verbrennungsraum 4 lässt sich auf diese Weise besonders effektiv unterstützen bzw. durchführen.

Durch die Pumpe 16 lässt sich die Probe 2 besonders schnell und vorteilhaft ansaugen, insbesondere, weil ein möglicher Strömungswiderstand im Verbrennungsraum 4 infolge eines dort befindlichen Katalysators kompensiert werden kann und eine besonders umfassende, quasi vollständige Absorption bzw. Adsorption im Sinn einer "Wiederfindung" gewährleistet werden kann.

Weiterhin vorzugsweise weist die Vorrichtung einen Amalgam-Kollektor 18 zum Binden bzw. Anreichern von in dem Verbrennungsraum 4 aus der Probe 2 freigesetztem Quecksilber auf. Der Amalgam-Kollektor 18 stellt in diesem Sinne eine "Amalgamfalle" dar. Der Amalgam-Kollektor 18 ist dabei vorzugsweise innerhalb der Gasströmungs-Führungseinrichtung 20 stromabwärts von dem Verbrennungsraum 4 angeordnet, beispielswiese zwischen dem Verbrennungsraum 4 und der Pumpe 16. Auf diese Weise lässt sich erzielen, dass die Pumpe 16 einen Transport des aus der Probe 2 stammenden Quecksilbers in den Amalgam-Kollektor 18 fördert oder bewirkt.

Wie an sich aus dem Stand der Technik bekannt, kann der Amalgam-Kollektor 18 dazu ausgebildet sein, Quecksilber festzuhalten bzw. anzureichern, das bei der Verbrennung der Probe 2 in dem Verbrennungsraum 4 freigesetzt worden ist. Das im Amalgam-Kollektor 18 gebundene Quecksilber kann dann durch Wärmezufuhr bzw. Ausheizen schlagartig freigesetzt und im Folgenden quantitativ erfasst werden, beispielsweise durch spektroskopisches Analysieren.

Der Verbrennungsraum 4 umfasst vorzugsweise einen ersten Heizbereich 41 und einen zweiten Heizbereich 42, wobei der erste Heizbereich 41 dazu ausgebildet ist, die Probe 2 zu verbrennen und der zweite Heizbereich 42 dazu ausgebildet ist, die bei dem Verbrennen der Probe 2 in dem ersten Heizbereich 41 entstandenen Verbrennungsgase zu zersetzen. Entsprechend der Abfolge der vorgesehenen Schritte bei der Durchführung einer Bestimmung des Quecksilbergehalts mit einer erfindungsgemäßen Vorrichtung ist dabei der zweite Heizbereich 42 vorteilhaft zwischen dem ersten Heizbereich 41 und der Gasströmungs-Führungseinrichtung 20 angeordnet.

Der erste Heizbereich 41 und der zweite Heizbereich 42 können also zusammen eine zweistufige Verbrennungszone bilden.

Wie bereits kurz erwähnt, kann der zweite Heizbereich 42 einen Katalysator aufweisen; dieser kann dafür vorgesehen sein, durch randseitige Umwandlung zu verhindern, dass - neben dem Quecksilber - Störparameter auf dem Amalgam des Amalgam-Kollektors 18 festgehalten werden.

Wie ebenfalls bereits kurz angedeutet, weist die Vorrichtung vorzugsweise weiterhin eine Analysevorrichtung 22 auf, die dazu ausgelegt ist, das in dem Verbrennungsraum 4 bei der Verbrennung der Probe 2 freigesetzte Quecksilber quantitativ zu erfassen. Bei der Analysevorrichtung 22 kann es sich beispielsweise um eine spektroskopische Vorrichtung handeln, die vorzugsweise eine Quecksilberlichtquelle 24 und einen dazugehörigen Detektor 26 mit einem Monochromator umfasst. Zwischen der Quecksilberlichtquelle 24 und dem dazugehörigen Detektor 26 kann eine Mess-Küvette angeordnet sein, die in die Gasströmungs-Führungseinrichtung 20 eingebunden ist und die dafür vorgesehen ist, von dem zu analysierenden Gas durchsetzt zu werden.

Die Analysevorrichtung 22 ist vorzugsweise stromaufwärts von der Pumpe 16 und stromabwärts von dem Amalgam-Kollektor 18 in der Gasströmungs-Führungseinrichtung 20 angeordnet.

Zwischen der Analysevorrichtung 22 und der Pumpe 16 ist dabei weiterhin vorteilhaft ein weiteres Ventilelement 28 in der Gasströmungs-Führungseinrichtung 20 angeordnet. Mit diesem lässt sich die Strömung des Gases innerhalb der Gasströmungs-Führungseinrichtung 20 gezielt beeinflussen. Insbesondere ist es mit dem Ventilelement 28 ermöglicht, statt einem Ansaugen mit der Pumpe 16 Gase, insbesondere Pyrolysegase - beispielsweise in Richtung eines Abzugs - freizusetzen.

Wie in Fig. 1 in dem ersten Heizbereich 41 skizziert, kann ein optional vorhandenes Schiffchen vorgesehen sein, das alternativ zur Probenzuführung mithilfe der zweiten Zuführungsvorrichtung 8 dazu verwendet werden kann, eine Probe in den Verbrennungsraum 4 einzuführen.

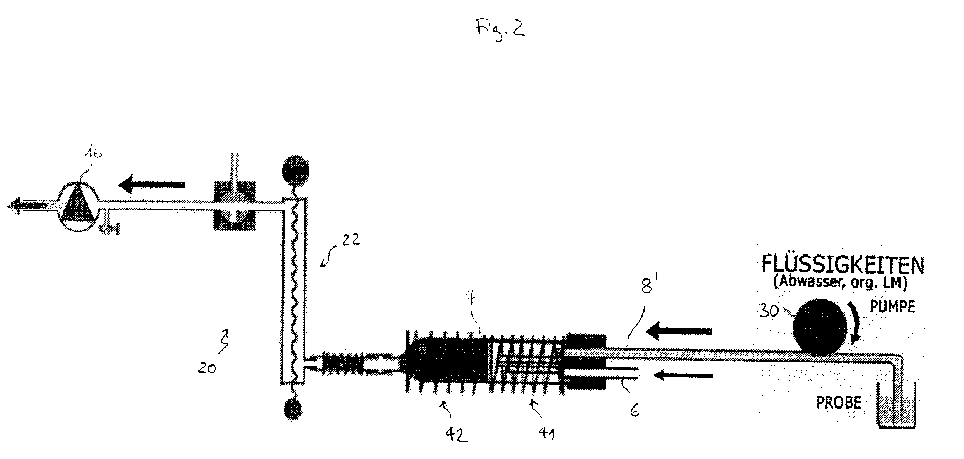

In Fig. 2 ist ein zweites Ausführungsbeispiel einer erfindungsgemäßen Vorrichtung prinzipiell skizziert. Im Folgenden wird lediglich auf Unterschiede zum ersten Ausführungsbeispiel eingegangen. Soweit nicht anders angegeben, gelten die obigen Ausführungen mit Bezug auf das erste Ausführungsbeispiel auch für das zweite Ausführungsbeispiel. Die Bezugszeichen sind analog verwendet.

Beim zweiten Ausführungsbeispiel ist die zweite Zuführungsvorrichtung 8' insbesondere dazu ausgebildet, eine flüssige Probe dem Verbrennungsraum 4 zuzuführen. Bei der flüssigen Probe kann es sich beispielsweise um Abwasser oder ein organisches Lösungsmittel handeln.

Die zweite Zuführungsvorrichtung 8' kann zur Zuführung der Probe ein Pumpelement 30 umfassen.

Das erfindungsgemäße Verfahren zur Bestimmung eines Quecksilbergehalts einer Probe 2 umfasst die folgenden Schritte:
(a) Zuführen von Sauerstoff über eine erste Zuführungsvorrichtung 6 in einen Verbrennungsraum 4,
(b) Zuführen der Probe 2 über eine zweite Zuführungsvorrichtung 8 in den Verbrennungsraum 4,
(c) Verbrennen der in Schritt (b) zugeführten Probe 2 in dem Verbrennungsraum 4 unter dem in Schritt (a) zugeführten Sauerstoff,
(d) Anreichern von Quecksilber, das bei Schritt (c) aus der Probe 2 freigesetzt worden ist,
(e) Quantitatives Erfassen des in Schritt (d) angereicherten Quecksilbers.

Insbesondere kann das Zuführen der Probe 2 in den Verbrennungsraum 4 durch eine andere Öffnung und/oder Zuleitung erfolgen als das Zuführen des Sauerstoffs in den Verbrennungsraum 4. Das Zuführen der Probe 2 in Schritt (b) kann entweder kontinuierlich oder diskontinuierlich erfolgen.

Es kann vorgesehen sein, dass eine gasförmige oder eine flüssige Probe 2 in den Verbrennungsraum 4 zugeführt wird.

Die erfindungsgemäße Vorrichtung ermöglicht eine Bestimmung eines Quecksilbergehalts für den Fall unterschiedlicher Proben, insbesondere gasförmiger und flüssiger Proben. Die Proben können kontinuierlich oder diskontinuierlich zugeführt werden. Bei den Proben kann es sich beispielsweise um brennbare Gase, Abgase, Pyrolysegase, Abwasser oder organische Lösungsmittel handeln. Die Vorrichtung bietet mithin einen besonders großen Anwendungsbereich.

## Patentansprüche

1. Vorrichtung zur Bestimmung eines Quecksilbergehalts einer Probe (2), aufweisend
- einen Verbrennungsraum (4) zum Verbrennen der Probe (2) unter Sauerstoff und
- eine erste, mit dem Verbrennungsraum (4) verbundene Zuführungsvorrichtung (6) zum Zuführen des Sauerstoffs in den Verbrennungsraum (4),
**gekennzeichnet durch**
- eine zweite, mit dem Verbrennungsraum (4) verbundene Zuführungsvorrichtung (8) zum Zuführen der Probe (2) in den Verbrennungsraum (4) und
- Mittel (16) zur Erzeugung eines Unterdrucks, die dazu ausgebildet sind, in dem Verbrennungsraum (4) befindliche Verbrennungsgase **durch** den Unterdruck aus dem Verbrennungsraum (4) zu befördern.

2. Vorrichtung nach Anspruch 1,
bei der die zweite Zuführungsvorrichtung (8) zum Zuführen einer flüssigen und/oder gasförmigen Probe in den Verbrennungsraum (4) ausgebildet ist.

3. Vorrichtung nach Anspruch 1 oder 2,
bei der die zweite Zuführungsvorrichtung (8) zum kontinuierlichen und/oder diskontinuierlichen Zuführen der Probe (2) in den Verbrennungsraum (4) ausgebildet ist.

4. Vorrichtung nach einem der vorhergehenden Ansprüche, weiterhin aufweisend
- eine Gasströmungs-Führungseinrichtung (20), die mit dem Verbrennungsraum (4) verbunden ist und die dazu ausgebildet ist, eine Gasströmung aus dem Verbrennungsraum (4) zu führen,
wobei die Mittel (16) zur Erzeugung des Unterdrucks, vorzugsweise in Form einer Vakuumpumpe, dazu ausgebildet sind, die im Verbrennungsraum (4) befindlichen Gase durch den Unterdruck in die Gasströmungs-Führungseinrichtung (20) zu saugen.

5. Vorrichtung nach Anspruch 4,
bei der die Pumpe (16) weiterhin dazu ausgelegt ist, die Probe (2) aus der zweiten Zuführungsvorrichtung (8) in den Verbrennungsraum (4) zu saugen oder eine Bewegung der Probe (2) aus der zweiten Zuführungsvorrichtung (8) in den Verbrennungsraum (4) zu unterstützen.

6. Vorrichtung nach einem der vorhergehenden Ansprüche, weiterhin aufweisend
- einen Amalgam-Kollektor (18) zum Binden von in dem Verbrennungsraum (4) aus der Probe (2) freigesetztem Quecksilber.

7. Vorrichtung mit den in den Ansprüchen 4 und 6 oder 5 und 6 genannten Merkmalen,
bei der der Amalgam-Kollektor (18) innerhalb der Gasströmungs-Führungseinrichtung (20) stromabwärts des Verbrennungsraums (4) angeordnet ist und die Mittel (16) zur Erzeugung des Unterdrucks dazu ausgebildet sind, die in dem Verbrennungsraum (4) befindlichen Verbrennungsgase vollständig aus dem Verbrennungsraum (4) in den Amalgam-Kollektor (18) zu saugen.

8. Vorrichtung nach einem der vorhergehenden Ansprüche, bei der der Verbrennungsraum (4) einen ersten Heizbereich (41) und einen zweiten Heizbereich (42) umfasst,
wobei der erste Heizbereich (41) zum Verbrennen der Probe (2) ausgebildet ist und der zweite Heizbereich (42) zum Zersetzen von bei dem Verbrennen der Probe (2) entstandenen Verbrennungsgasen ausgebildet ist.

9. Vorrichtung nach einem der vorhergehenden Ansprüche, bei der der Verbrennungsraum (4) durch eine Heizvorrichtung gebildet ist, die ein beheizbares Rohr aus Quarz oder Keramik umfasst.

10. Vorrichtung nach einem der vorhergehenden Ansprüche, weiterhin aufweisend
- eine Analysevorrichtung (22) zur quantitativen Erfassung des in dem Verbrennungsraum (4) bei der Verbrennung aus der Probe (2) freigesetzten Quecksilbers.

11. Vorrichtung mit den in den Ansprüchen 4 und 10 oder 5 und 10 genannten Merkmalen,
bei der die Analysevorrichtung (22) innerhalb der Gasströmungs-Führungseinrichtung (20) stromabwärts des Verbrennungsraums (4) angeordnet ist.

12. Vorrichtung nach Anspruch 10 oder 11,
bei der die Analysevorrichtung (22) eine spektroskopische Vorrichtung ist, die vorzugsweise eine Quecksilberlichtquelle und einen dazugehörigen Detektor mit einem Monochromator umfasst.
